# EUROPEAN PATENT SPECIFICATION

(11) **EP 1 141 007 B1**
(45) Date of publication and mention of the grant of the patent: **15.05.2013**
(21) Application number: 99964259.8
(22) Date of filing: 14.12.1999
(51) Int. Cl.: C07K 14/47, A61K 39/00

(54) **COMPOSITIONS AND METHODS FOR ENHANCEMENT OF MAJOR HISTOCOMPATIBILITY COMPLEX CLASS I RESTRICTED ANTIGEN PRESENTATION**
ZUSAMMENSETZUNGEN UND METHODEN ZUR STEIGERUNG DER HAUPTHISTOKOMPATIBILITÄTSKOMPLEX KLASSE I ABHÄNGIGEN ANTIGEN-PRÄSENTIERUNG
COMPOSITIONS ET PROCEDES D'AMELIORATION DE LA PRESENTATION D'ANTIGENE RESTREINTS DU COMPLEXE MAJEUR D'HISTOCOMPATIBILITE DE CLASSE I

(30) Priority: 14.12.1998 US 112324 P
(43) Date of publication of application: 10.10.2001
(73) Proprietor: DENDREON CORPORATION, Seattle, WA 98121 (US)
(72) Inventor: LAUS, Reiner, San Carlos, CA 94070 (US); HAKIM, Itzhak, Palo Alto, CA 94306 (US); VIDOVIC, Damir, Mountain View, CA 94043 (US)
(74) Representative: Vossius & Partner
(86) International application number: PCT/US1999/029724
(87) International publication number: WO 2000/035949

(56) References cited:
- EP-A- 0 497 997
- WO-A-96/10038
- WO-A-96/12009
- LAYTON G T ET AL: "Induction of HIV-specific cytotoxic T lymphocytes in vivo with hybrid HIV-1 V3:Ty-virus-like particles." JOURNAL OF IMMUNOLOGY, (1993 JUL 15) 151 (2) 1097-107. , XP000887242
- BUSCHLE M ET AL: "Transloading of tumor antigen -derived peptides into antigen - presenting cells." PROCEEDINGS OF THE NATIONAL ACADEMY OF SCIENCES OF THE UNITED STATES OF AMERICA, (1997 APR 1) 94 (7) 3256-61. , XP002117665
- LAUS R ET AL: "ENHANCED MAJOR HISTOCOMPATIBILITY COMPLEX CLASS I-DEPENDENT PRESENTATION OF ANTIGENS MODIFIED WITH CATIONIC AND FUSOGENIC PEPTIDES", NATURE BIOTECHNOLOGY, NATURE PUBLISHING GROUP, NEW YORK, NY, US, vol. 18, no. 12, 1 December 2000 (2000-12-01), pages 1269-1272, XP001079887, ISSN: 1087-0156, DOI: 10.1038/82377

### SEQUENCE LISTING

<110> Dendreon Corporation
<120> Compositions and Methods for Enhancement of Major Histocompatibility Complex Class I Restricted Antigen Presentation
<130> 7636-0020.41
<140> Not Yet Assigned
   <141> Filed Herewith
<150> US 60/112,324 <151> 1998-12-14
<160> 7
<170> FastSEQ for Windows Version 4.0
<210> 1
   <211> 21
   <212> PRT
   <213> Artificial Sequence
<220>
   <223> peptide
<400> 1
   Cys Lys Lys Lys Lys Lys Lys Lys Lys Lys Lys Lys Lys Lys Lys Lys
    1               5                  10                  15
   Lys Lys Lys Lys Lys
               20
<210> 2
   <211> 25
   <212> PRT
   <213> Artificial Sequence
<220>
   <223> peptide
<400> 2
   Cys Glu Ala Ala Ala Ala Ala Glu Ala Ala Ala Ala Ala Glu Ala Ala
    1              5                   10                  15
   Ala Ala Ala Glu Ala Ala Ala Ala Ala
               20                  25
<210> 3
   <211> 24
   <212> PRT
   <213> Artificial Sequence
<220>
   <223> peptide
<400> 3
   Cys Gly Leu Phe Gly Ala Ile Ala Gly Phe Ile Glu Asn Gly Trp Glu
    1              5                   10                  15
   Gly Met Ile Asp Gly Trp Tyr Gly
                                   20
<210> 4
   <211> 45
   <212> PRT
   <213> Artificial Sequence
<220>
   <223> peptide
<400> 4
   Cys Glu Ala Ala Ala Ala Ala Glu Ala Ala Ala Ala Ala Glu Ala Ala
    1              5                   10                  15
   Ala Ala Ala Glu Ala Ala Ala Ala Ala Lys Lys Lys Lys Lys Lys Lys
               20                  25                  30
   Lys Lys Lys Lys Lys Lys Lys Lys Lys Lys Lys Lys Lys
           35                  40                  45
<210> 5
   <211> 44
   <212> PRT
   <213> Artificial Sequence
<220>
   <223> peptide
<400> 5
   Cys Gly Leu Phe Gly Ala Ile Ala Gly Phe Ile Glu Asn Gly Trp Glu
    1              5                   10                  15
   Gly Met Ile Asp Gly Trp Tyr Gly Lys Lys Lys Lys Lys Lys Lys Lys
               20                  25                  30
   Lys Lys Lys Lys Lys Lys Lys Lys Lys Lys Lys Lys
           35                  40
<210> 6
   <211> 21
   <212> PRT
   <213> Artificial Sequence
<220>
   <223> peptide
<221> VARIANT
   <222> (1)...(21)
   <223> Xaa = Arg or Lys
<400> 6
   Cys Xaa Xaa Xaa Xaa Xaa Xaa Xaa Xaa Xaa Xaa Xaa Xaa Xaa Xaa Xaa
    1               5                  10                  15
   Xaa Xaa Xaa Xaa Xaa
               20
<210> 7 - -
   <211> 20
   <212> PRT
   <213> Artificial Sequence
<220>
   <223> peptide
<221> VARIANT
   <222> (1)...(20)
   <223> Xaa = Arg or Lys
<400> 7
   Xaa Xaa Xaa Xaa Xaa Xaa Xaa Xaa Xaa Xaa Xaa Xaa Xaa Xaa Xaa Xaa
    1               5                  10                  15
   Xaa Xaa Xaa Xaa
               20

## Description

The present invention relates to compositions of soluble protein antigens (Ag) which have been modified to render them presentable in the context of major histocompatibility complex class I molecules (MHC class I).

Tumor specific CD8⁺ cytotoxic T lymphocytes (CTL) appear to constitute an important effector limb of the anti-tumor immune response as indicated by animal model studies (Greenberg, 1991, Adv. Immunol. 49: 281). Therefore, tumor specific Ag recognized by CTL are likely to function as tumor rejection Ag, capable of inducing protective immunity *in vivo*.

MHC class I and class II molecules, constituitively expressed on APC, are responsible for the presentation of non-overlapping Ag-derived peptides to CD8⁺ CTL and CD4⁺ helper T cells (Th), respectively (Rothbard et al., 1937, Nature 326: 881; Babbitt et al., 1985, Nature 317: 359). CTL recognize class I molecules containing peptidic fragments of intracellular proteins that have been transported into endoplasmic reticulum prior to their transfer to the MHC molecule (Germain, 1995, Ann. NY Acad. Sci. 754:114; Heemels & Ploegh, 1995, Annu. Rev. Biochem. 64:463), while the bulk of class II completed peptides presented to Th cells are degradation products of exogenous or cell surface proteins that enter the biosynthetic pathway of class II molecules via endocytosis and a subsequent fusion with lysosomes (Cresswell, 1994, Annu. Rev. Immunol. 12: 259). This dichotomy of Ag presentation explains why Ag-specific CD8⁺ CTL are generated most effectively against intracellular Ag, or when the extracellular Ag are delivered into the cytosol. Since intact proteins in the extracellular medium do not ordinarily penetrate into the cytosol, soluble proteins typically fail to elicit CTL responses (Braciale et al., 1987, Immunol. Rev. 98: 95).

It is therefore desirable to provide a method for eliciting CTL responses against soluble proteins.
Layton G. T. et al., 1993, Journal of Immunology, 151 (2) 1097 discloses a hybrid composition V3:Ty-VLP consisting of Ty virus-like carrying the V3 region of HIV-1 gp120/160 envelope protein which induces V3-specific CTL in mice and also facilitates uptake into APC with subsequent access to the MHC class I processing pathway.
Buschle M. et al., 1997, Proceedings of the National Academy of Sciences of the United States of America, 94 (7) 3256 discloses the transloading of tumour antigen-derived peptides into APC using polycationic amino acids (polyarginine or polylysine) which enhance the delivery as compared with cells treated with peptide alone.
WO 96/12009 discloses methods for engineering APCs for producing an APC having a defined MHC:nominal antigen or costimulator on its membrane.
The present invention provides an antigen composition which elicits an enhanced cytotoxic T cell response in the context of a major histocompatibility complex class I molecule (MHC class I), comprising a soluble protein antigen (i) chemically linked or (ii) conjugated by expression of a continuous nucleic acid coding sequence to one or more added peptidic sequences selected from the group consisting of SEQ ID NO:2, SEQ ID NO:3, SEQ ID NO:4, and SEQ ID NO:5 and a sequence presented as CYS-[X-Y-Y-Y-Y-Y]ₙ; wherein X = glu or asp, Y = ala, leu, ile, phe, gly, cys, met or val and n is greater than or equal to 3 or [X-Y-Y-Y-Y-Y]ₙ; wherein X = glu or asp, Y = ala, leu, ile, phe, gly, cys, met or val and n is greater than or equal to 3, wherein said added peptidic sequence facilitates entry of said antigen into antigen presenting cells (APC), and wherein said antigen composition activates T cells to produce a cytotoxic cellular immune response against said antigen, at a T cell level that is higher than that produced by such APC stimulated by the antigen alone.

In some cases, the added peptidic sequence is a peptide having about 20 to 25 amino acid residues which may or may not have an N-terminal cysteine residue.

The invention also provides an antigenic composition for *in vivo* immunotherapy of a mammal, comprising one or more modified antigens, each having an added peptidic sequence.

In one aspect, the one or more modified protein antigens of the invention are specific to a given tumor er pathogen and may be used for treatment of a malignant tumor in a mammal.

Also described is an immunization method, *e.g*., for cancer therapy, wherein the method includes the steps of obtaining a sample of DC from a subject, exposing the DC to a modified soluble protein antigen *in vitro* in a manner effective to induce cell-surface presentation of one or more peptide antigens against which an immune response is desired, and returning the exposed and stimulated DC to the subject.

These and other objects and features of the invention will become more fully apparent when the following detailed description is read in conjunction with the accompanying drawings.

### Brief Description of the Drawings

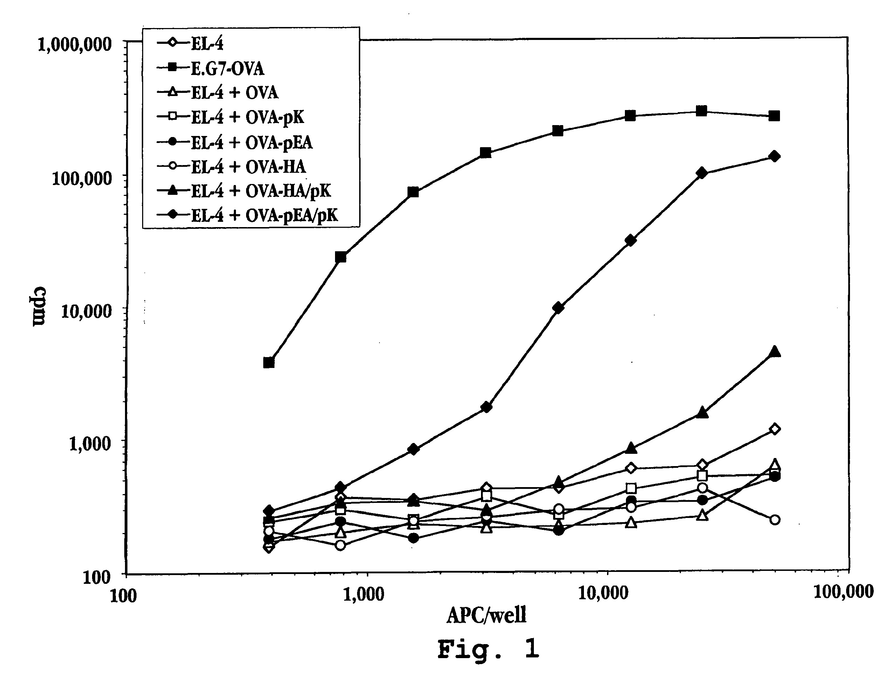

Figure 1 shows the responses of interleukin-2 (IL-2) secreting mouse T cell hybridoma B3Z (Jameson et al., 1993, J. Exp. Med. 177: 1541) to various forms of ovalbumin (OVA) presented by the syngeneic APC line, EL-4. Cpm refers to counts per minute; HA refers to influenza virus hemagglutinin derived peptide, OVA refers to native ovalbumin; OVA-HA refers to the HA-conjugated form of OVA; OVA-pEA refers to the pEA-conjugated form of OVA; OVA-pK refers to the pK-conjugated form of OVA; OVA-HA/pK, refers to the HA and pK-conjugated form of OVA; and OVA-pEA/pK refers to the pEA and pK-conjugated form of OVA. E.G7-OVA is the EL-4 cell line transfected with full length OVA cDNA (Brossart et al., J. Immunol. 158: 3270, 1997).

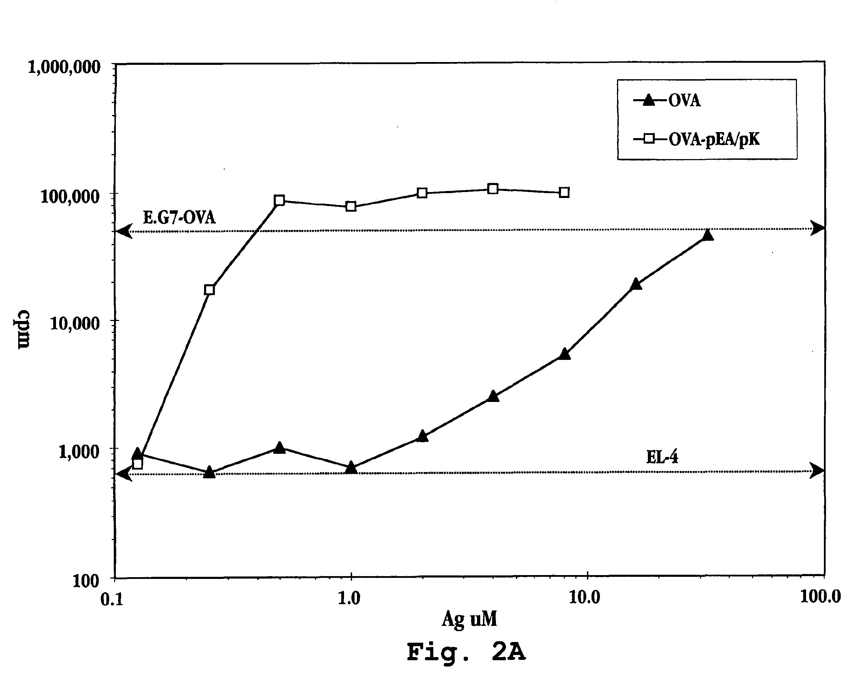

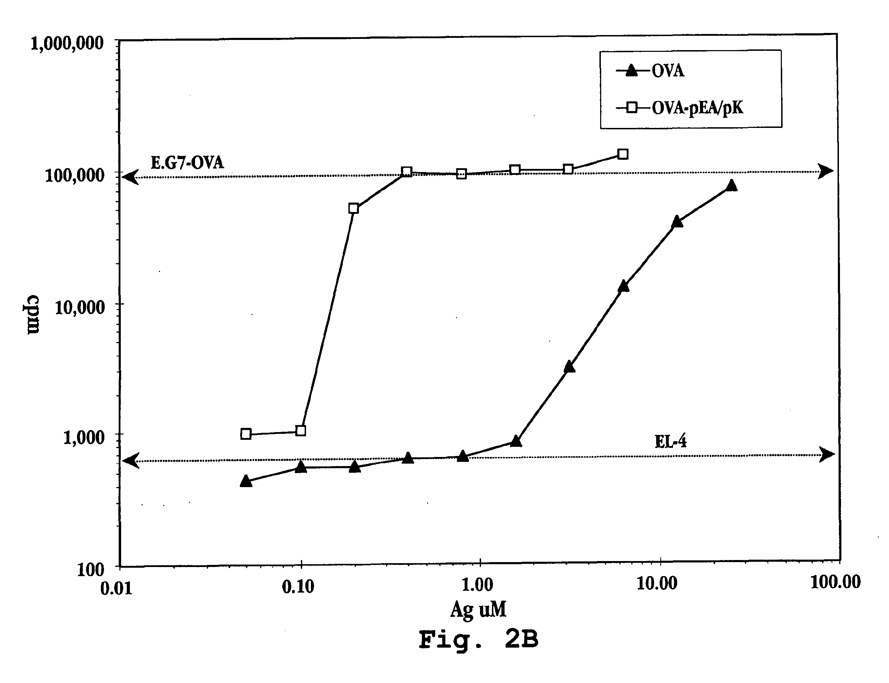

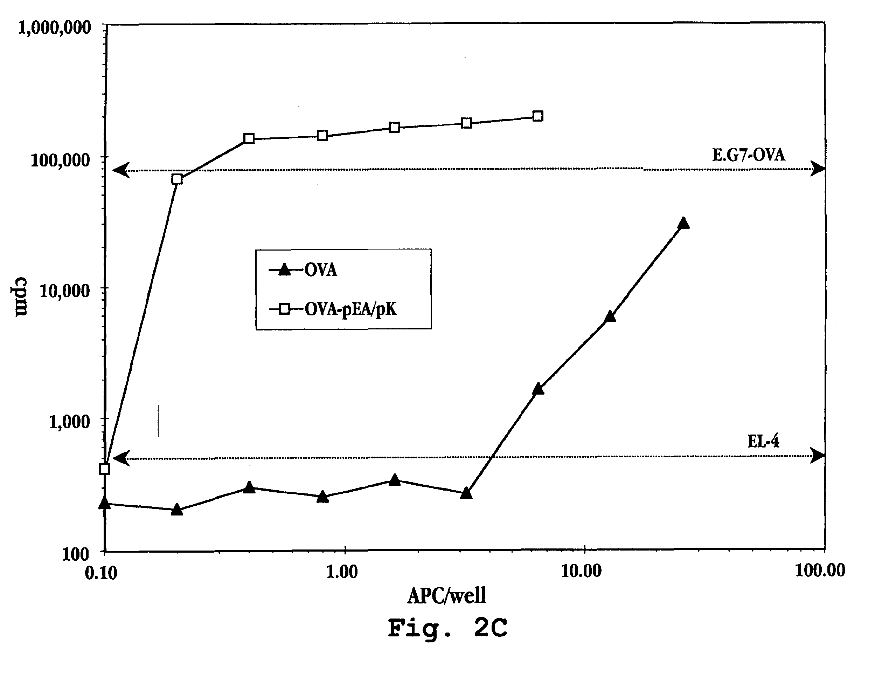

Figures 2A-C show the response of the B3Z T cell hybridoma to EL-4 APC pre-pulsed with OVA or OVA-pEA/pK preparations at different Ag concentrations. Fig. 2A, 2B and 2C each represent an independent experiment. In each case cpm represents cell growth measured by incorporation of [³H]thymidine present during the final 6 h culture period. The "EL-4" and "E.G7-OVA" lines indicate the level of B3Z response to the negative and positive control APC, respectively, in the absence of additional soluble Ag.

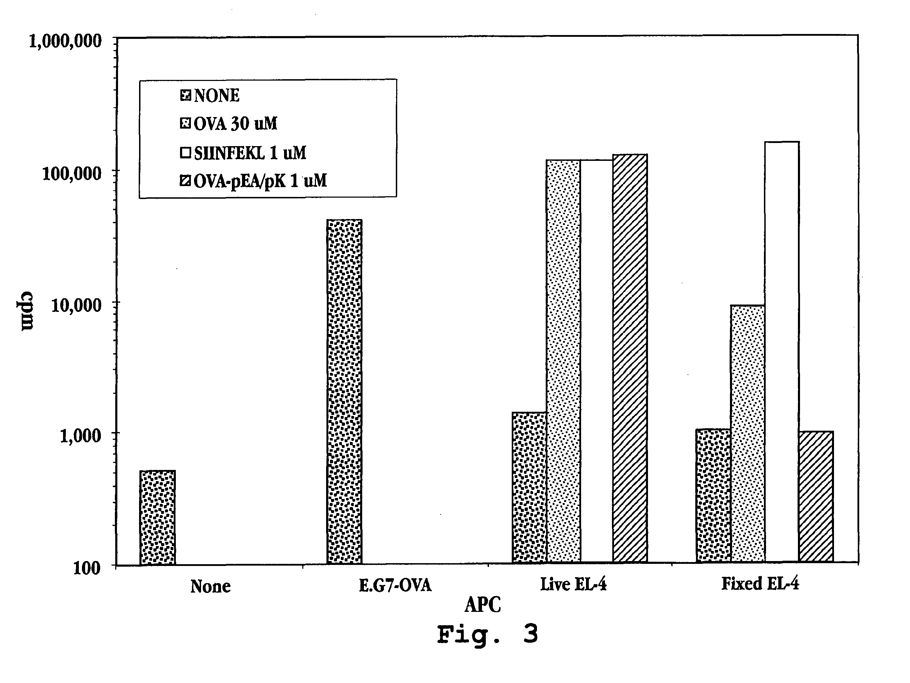

Figure 3 shows the response of B3Z T cell hybridomas to APC pre-pulsed with SIINFEKL, or OVA or OVA-pEA/pK at their respective optimal concentrations. Cpm corresponds to cell growth as described above for Figs. 2A-C.

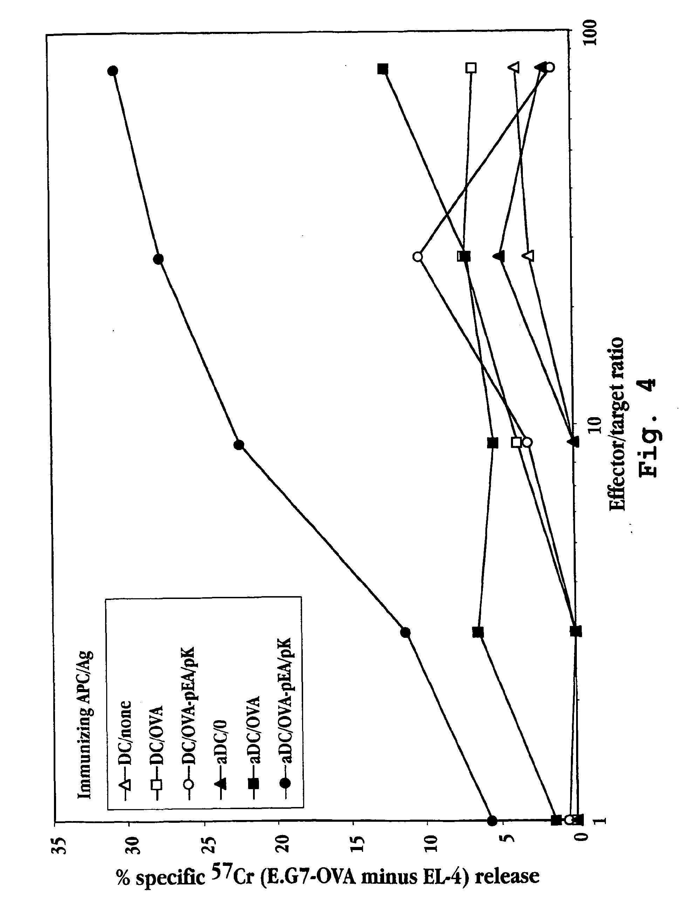

Figure 4 shows the class I restricted OVA specific CTL responses of mice pre-immunized with Ag-pulsed DC. The enriched DC were prepared, pulsed with either OVA (5 µM) or OVA-pEA/pK (0.5 µM), and injected into mice as described in Example 2. Six days later the cultures were tested for their ability to lyse ⁵¹Cr-labeled E.G7-OVA and EL-4 target cells in a standard cell mediated cytotoxicity assay.

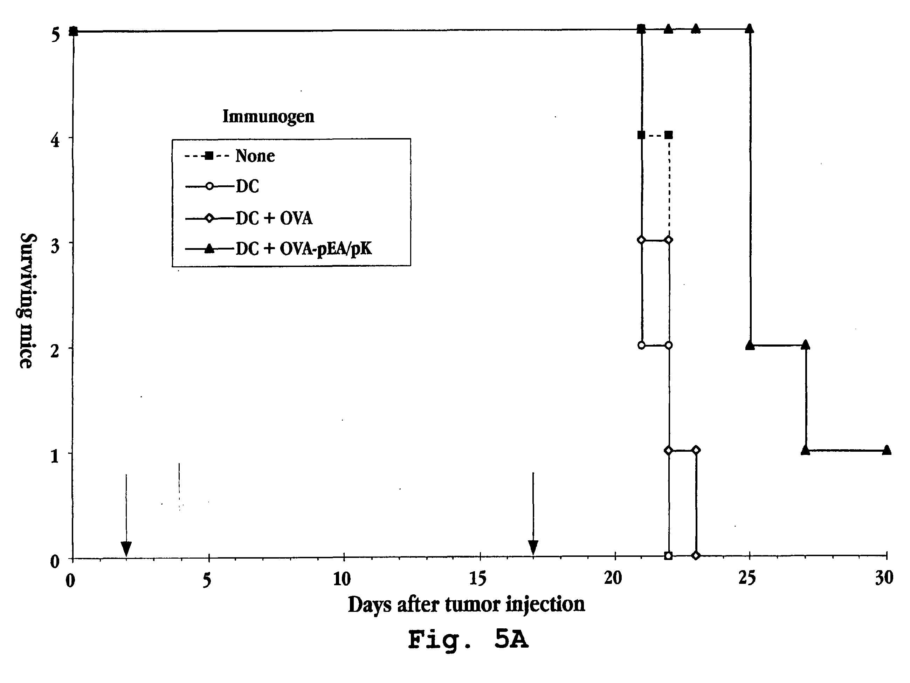

Figures 5A-B show the survival of E.G7-OVA injected mice treated with Ag-pulsed APC. Twenty 8-week old randomized female C57BU6 mice were injected i.p. with 25 x 10⁶ and 2 x 10⁶ E.G7-OVA cells in 0.1 ml PBS (Fig. 6A and Fig. 6B, respectively). Two days and again 2 weeks later (arrows), mice received i.p. injections of DC, OVA-pulsed DC, OVA-pEA/pK-pulsed DC (5 x 10⁵ cells per 0.1 ml injection), or PBS. Mice were monitored daily and their survival was recorded as indicated.

### I. Definitions

Unless otherwise indicated, the terms below have the following meanings:
As used herein, "presentation of soluble protein antigens in the context of major histocompatibility complex class I molecules (MHC I)" means the soluble protein antigen or fragments thereof, are displayed together with major histocompatibility complex class I molecules on the cell surface and can serve as an inducer and target of class I restricted antigen-specific CTL.

As used herein, the term "pulse" means exposure of APC to antigen for a time sufficient to promote presentation of that antigen on the surface of the APC. APC which have been pulsed and present antigen on the APC surface are said to be "stimulated".

"Precursor antigen presenting cells" are cells that when exposed to an Ag or peptide are capable of becoming APC and presenting Ag to T cells.

As used herein, "antigen presenting cells" (APC) are any cells which, after being pulsed with Ag or peptide, can activate CD8⁺ cytotoxic T-lymphocytes (CTL) or CD4⁺ helper T-lymphocytes in an immune response.

"Dendritic cell precursors", or "DCP", are peripheral blood cells which can mature into DC under suitable conditions. DCP typically have a non-dendritic morphology and are not competent to elicit a primary immune response as antigen presenting cells.

"Dendritic cells", or "DC" are matured DCP, which typically have a dendritic cell morphology, that is, they are large veiled cells which extend dendrites when cultured *in vitro.* When pulsed with Ag or peptide, such DC are capable of presenting Ag to naive T cells.

As used herein, the term "modified antigen presenting cells" (modified APC) refers to a population of APC which have been treated *ex vivo* such that they have an enhanced ability to present antigen in the context of MHC class I relative to APC which have not been modified.

Similarly, as used herein, the term "modified dendritic cells" (modified DC) refers to a population of DC which have been treated *ex vivo* such that they have an enhanced ability to present antigen in the context of MHC class I relative to DC which have not been modified.

The term "more effectively" when used herein relative to the presentation of soluble proteins antigens means at least a 5-fold increase in the magnitude of detectable T cell response following presentation of a soluble protein antigen by APC. For example, in one case this means at least a 5 fold increase in the number of responder cells is detected following presentation of a given antigen by a designated number of APC relative to the number of detectable responder cells obtained when the same number of APC from a reference population present the same antigen under the same culture conditions. In another case, this means that at least a 5-fold increase in the magnitude of T cell response is detected following presentation of a given antigen by a designated number of APC relative to magnitude of T cell response obtained when a different or modified antigen is presented by the same number of APC under the same culture conditions and at an equimolar Ag concentration.

As used herein, the terms "tumor" and "cancer" are used interchangeably and refer to a cell that exhibits a loss of growth control and forms unusually large clones of cells. Tumor or cancer cells generally have lost contact inhibition and may be invasive and/or have the ability to metastasize.

### II. Immune Response to Soluble Protein Antigens

In experimental systems, tumor antigen specific cytotoxic T lymphocytes (CTL) are the most powerful immunological mechanism for the elimination of tumors. CTL can be induced either *in vivo* with vaccines or can be generated *in vitro* and then be re-infused into the tumor-bearing organism. The *in vivo* induction of CTL is typically accomplished by immunization with live virus or cells (Tanaka, et al., J. Immunol., (1991), 147, 3646-52, Wang, et al., J. Immunol., (1995), 4685-4692).

Except for a few special viral proteins such as the SV-40 large T antigen and the Hepatitis B surface antigen, injection of isolated or soluble proteins does not result in induction of CTL (Schirmbeck, et al., Eur. J. Immunol., (1993), 23, 1528-34). CTL are induced when a protein enters the major histocompatibility complex class I ("MHC I" or "class I") pathway of antigen processing. To enter this pathway the protein must be present in the cytosol of an antigen presenting cell (APC). There it is degraded into peptides which are then transported into the endoplasmic reticulum, where they associate with HLA class I molecules. These peptides are then displayed together with the class I molecules on the cell surface and can serve as an inducer and target of class I restricted antigen-specific CTL. Physiologically, only proteins that are endogenously synthesized by the APC enter this pathway.

The priming of an immune response expands and activates "naive" lymphocytes, *i.e*., those that have not previously seen an immunogen to become "effector" cells that actively respond. Each naive cell has the potential for seeing one and only one antigenic epitope, a situation analogous to a key fitting into a lock. Only those cells that recognize their cognate epitope become effector cells.

T-cells can be of the "helper" or "cytotoxic" (cytotoxic) type. Helper T cells secrete growth factors for lymphoid cells that stimulate the activation and function of B and T cells. The cytotoxic T cells recognize and either directly, or indirectly, kill cells that express a particular antigen. Like B cells, each T cell has receptors specific for one and only one antigenic epitope. T cell receptors recognize fragments of proteins that are displayed on the cell surface by major histocompatibility complexes (MHC).

There are two different types of MHC proteins, Class I and Class II, both of which present proteolytically degraded fragments of proteins to T cells. Class I molecules which are expressed on most cells of the body and present fragments of endogenously synthesized proteins to cytotoxic T cells. Class II molecules which are expressed on specialized antigen presenting cells (APCs) such as macrophages, monocytes, dendritic cells and B cells present protein fragments to T helper cells. (Chen, CH and Wu, TC, J Biomed Sci., 5(4):231-52 1998)

In most cases, Class I molecules present foreign proteins synthesized in a cell. For presentation by Class II, the foreign protein either can be synthesized in the cell or taken up by the cell from the outside (*i.e.,* presented in the form of a free protein or peptide). If an antigen is synthesized in a cell and presented by both Class I and Class II molecules, both antibody producing B cells and cytotoxic T cells are produced. However, if an antigen originated outside of a cell and is expressed only by Class II, the specific immune response is largely limited to T helper cells and antibody production. [THE SCIENTIFIC FUTURE OF DNA FOR IMMUNIZATION, American Academy of Microbiology, Robinson, et al., Eds., 1-29, 1997]

Accordingly, the typical response to soluble protein antigens is a Glass II mediated response. The present invention represents compositions and methods which allow soluble protein antigens to enter the Class I pathway which is typically reserved for foreign cellular antigens.

In addition, some progeny of antigen-stimulated T cells do not develop into effector cells, but become memory cells that are capable of surviving for long periods of time in the absence of additional antigenic challenge. Such memory cells are quiescent and do not produce effector molecules unless they are stimulated by antigen. (See, e.g., Abbas, AK et al., Eds. CELLULAR AND MOLECULAR IMMUNOLOGY, W.B. Saunders Co., pages 116-123; 130-134, 1997)

Naïve T cells (or T cells that have not been previously exposed to a given antigen) require only the correct MHC I-restricting molecule to survive, however to expand, they also must be exposed to antigen. In contrast, memory T cells have a lower functional activation threshold that facilitates secondary responses which are more rapid and stronger than that of naïve T cells.

### III. Modified Soluble Protein antigen Compositions

The present invention is based on the discovery that when peptidic sequences such as those presented as pE A (SEQ ID NO:2), are covalently linked to an antigen, the antigen is capable of triggering naive CTL responses *in vivo,* and is about 100 fold more efficient in stimulating corresponding class I restricted memory T cells *in vitro.*

The added peptidic sequence generally comprises a peptide having about 20 to 25 amino acid residues.

The peptides are presented as CYS-[X-Y-Y-Y-Y-Y]ₙ; wherein X = glu or asp, Y = ala, leu, ile, phe, gly, cys, met or val and n is greater than or equal to 3 (designated sequence 8), with a specific example provided by the pEA peptide presented as SEQ ID NO:2.

The modified soluble protein antigen may be chemically linked to one or more peptidic sequences or may be formed by expression of a continuous nucleic acid coding sequence comprising one or more of said peptidic sequences, as exemplified by SEQ ID NO:4 and SEQ ID NO:5.

When such peptidic sequences are chemically linked to one or more other peptidic sequences, the sequences may or may not have an N-terminal cysteine.

In a preferred embodiment, the present invention provides an antigen composition for *in vivo* administration comprising one or more soluble protein antigens covalently conjugated to peptides selected from the group consisting of the pK, pEA, HA. tandem pEA/pK, tandem HA/pK peptides, presented as SEQ ID NO:1, SEQ ID NO:2, SEQ ID NO:3, SEQ ID NO:4,and SEQ ID NO:5, respectively (Table 1).

In a preferred embodiment, the one or more soluble protein antigens is covalently conjugated to the pEA peptide (SEQ ID NO: 2), such that the conjugate enters the class I MHC presentation pathway, consequently stimulating antigen specific CD8⁺ CTL.

Modified soluble protein antigens having added peptidic sequences can also be formed recombinantly is fusion proteins according to methods known in the art, as further described below.

An exemplary application of the antigenic compositions of the present invention is the use of pEA-conjugated form of an antigen which is expressed by and isolated from tumor cells, in an antigenic composition for administration to a cancer patient (*i.e*., a "vaccine"). Such an antigenic composition can provide effective immunotherapy for various tumors, such as, but not limited to prostate and breast carcinoma, multiple myeloma, etc.

As shown in Example 2, this treatment is particularly effective when the pEA peptide (SEQ ID NO: 2) is conjugated to an antigen expressed by and isolated from tumor cells which is co-cultured with DC to activate them *ex vivo,* followed by administration to a subject.

### IV. Production of recombinant fusion proteins

Modified soluble protein antigens having one or more added peptidic sequences may also be produced recombinantly as fusion proteins by expression of continuous nucleic acid coding sequences according to methods known in the art.

Unless otherwise indicated, all terms used herein have the same meaning as they would to one skilled in the art of the present invention. Practitioners are particularly directed to Sambrook et al. (1989) MOLECULAR CLONING: A LABORATORY MANUAL (Second Edition), Cold Spring Harbor Press, Plainview, N.Y. and Ausubel FM et al. (1989) CURRENT PROTOCOLS IN MOLECULAR BIOLOGY, John Wiley & Sons, New York, N.Y., for definitions and techniques routinely used by those of skill in the art.

The invention includes modified soluble antigen fusion proteins produced using recombinant techniques. Such fusion proteins are produced by culturing recombinant prokaryotic or eukaryotic host cells comprising nucleic acid sequences encoding the fusion protein under conditions promoting expression of the fusion proteins, followed by recovery of the modified soluble antigen fusion proteins from the host cells or the cell culture medium.

The nucleic acid coding sequence for the fusion protein may be inserted into any one of a variety of expression vectors for expressing a polypeptide, as long as it is replicable and viable in the host. In general, DNA is inserted into an appropriate restriction endonuclease site(s) using routine techniques. Such procedures and related sub-cloning procedures are deemed to be within the scope of those skilled in the art. The vector may comprise regulatory sequences, including, for example, non-coding sequences, such as introns and control elements, i.e., promoter and terminator elements or 5' and/or 3' untranslated regions, effective for expression of the coding sequence in a suitable host and/or in a vector or host environment in which the modified soluble protein antigen coding sequence is not normally expressed, operably linked to the coding sequence. Large numbers of suitable vectors and promoters are known to those of skill in the art, are commercially available and are described in Sambrook, *et al.,* (*supra*).

The present invention also relates to host cells which are genetically engineered with vectors useful to produce the modified soluble antigen fusion proteins of the invention by recombinant techniques. Host cells are genetically engineered (*i.e*., transduced, transformed or transfected) with an appropriate vector which may be, for example, a cloning or expression vector: The vector may take the form of a plasmid, a viral particle, a phage, *etc*. The culture conditions, such as temperature, pH and the like, are those previously used with the host cell selected for expression, and will be apparent to those skilled in the art.

Methods of introducing nucleic acids into cells for expression of heterologous proteins are also known to the ordinarily skilled artisan, for example, calcium phosphate transfection, DEAE-Dextran mediated transfection, electroporation, nuclear microinjection, bacterial protoplast fusion with intact cells, or use of polycations, *e.g*., polybrene, polyornithine, may be used. General aspects of transformation involving mammalian cells have been described in U.S. Patent No. 4,399,216, and Keown et al., Methods in Enzymology, 185:527-537 (1990).

Suitable host cells for cloning or expressing the DNA in the vectors herein include prokaryote, yeast, or higher eukaryotic cells. Suitable prokaryotes include but are not limited to eubacteria, such as gram-negative or gram-positive organisms, for example, *E. coli.*

Suitable host cells for the expression of glycosylated soluble antigen fusion proteins are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. The selection of the appropriate host cell is deemed to be within the skill in the art.

A process for producing such soluble antigen fusion proteins comprises culturing host cells under conditions suitable for expression of the fusion protein and recovering the fusion protein from the cell culture. In general, principles, protocols, and practical techniques for maximizing the productivity of cell cultures can be found in MAMMALIAN CELL BIOTECHNOLOGY: A PRACTICAL APPROACH, M. Butler, ed. (IRL Press, 1991) and Sambrook *et al., supra.* More specifically, techniques for expression in the Baculovirus system are described in Engelhard EK et al. Proc. Nat. Acad. Sci. 91:3224-3227, 1994.

Host cells transformed with nucleotide sequences encoding the soluble antigen fusion proteins of the invention may be cultured under conditions suitable for the expression and recovery of the encoded protein from cell culture. The protein produced by a recombinant cell may be secreted, membrane-bound, or contained intracellularly depending on the sequence and/or the vector used.

As understood by those of skill in the art, expression vectors containing polynucleotides encoding the modified soluble protein antigens of the invention can be designed with signal sequences which direct secretion of such modified antigens through a prokaryotic or eukaryotic cell membrane.

The modified soluble proteins antigens of the invention whether produced by chemical coupling or by expression of continuos coding sequences as recombinant fusion proteins may be used to pulse APC, and be presented by such APC in the context of MHC I.

### V. Antigen Presenting Cells

### A. Enrichment of DC

Although exemplified methods include cell adherence to tissue culture plastic, which achieves the enrichment of DC, it will be understood that other approaches may be used to obtain such a cell fraction. For example, combined density gradient centrifugation and affinity cell separation may be used.

DC have been isolated and purified using a variety of methodologies incorporating, for example, multiple-step density-gradient based isolation, monoclonal antibody panning, depletion of lineage positive cells and serum-supplemented cultures (Macatonia, S.E., et al., Immunology 74:399-406, 1991; Markowicz, S., and Engleman, E.G., J. Clin. Invest. 85:955-961, 1990; Young, J.W., and Steinman, R.M., Cell. Immunol. 111:167-182, 1987).

In one exemplary method, DC are obtained from peripheral blood by performing the steps of (1) standard leukapheresis; (2) buoyant density centrifugation either with a one-step or successive two-step procedure; and (3) culture of the cells *ex vivo* in serum free medium for 40 hours, in the absence of exogenously added cytokines. If desired, DC can be further enriched by (4) post-culture enrichment by buoyant density centrifugation; and (5) depletion of monocytes/macrophages and B lymphocytes using CD14 and CD19 immunomagnetic beads. (See, e.g., co-owned, copending USSN 60/087,764.)

The DC so isolated are characterized by their dendritic morphology and by a phenotype that is positive for surface expression of HLA-DR (human leukocyte antigen, DR) and negative for surface antigens specific to particular cell lineages (*i.e.,* CD3, CD14, CD16, CD19, CD20 and CD56 negative; designated herein as , "lin -"), and the ability to elicit primary and secondary immune responses when co-cultured with human lymphocytes. (See, e.g., co-owned, copending USSN 60/158,618.)

### B. Evaluation of antigen presentation

An antigen presentation assay is used to evaluate the antigen presenting ability of APC (or DC) and to evaluate the presentation of various antigens. An exemplary assay is described in Example 1, wherein APC (exemplified by EL4 cells), are pulsed with antigen, followed by antigen presentation by the APC to responder cells (exemplified by B3Z cells), with an evaluation of responder proliferation by ³H incorporation. The assay format described herein may be used to evaluate the antigen presenting ability of APC by using a fixed amount of antigen in the pulsing step together with a titration of APC or the addition of a fixed number of APC subjected to various treatments to activate them. Alternatively, the assay format may be used to evaluate the ability of various antigen to be presented by using a titration of the antigen together with a fixed number of APC. In each case, the endpoint is a measurement of the corresponding proliferation of responder cells as indicated by ³H incorporation.

As described in Example 1, when EL-4 APC were pulsed with a conjugate comprising the pK peptide (SEQ ID NO: 1) and the pEA peptide (SEQ ID NO: 2), or the pK peptide (SEQ ID NO: 1) and the HA peptide (SEQ ID NO: 3) linked to the OVA antigen at a concentration equivalent to the nonstimulatory (*i.e.,* suboptimal) dose of native OVA, both conjugates triggered positive responses. Among the two, the OVA-pK/pEA conjugate was more potent.

When EL-4 APC were pre-pulsed with a wide range of antigen concentrations, it was observed that the pEA/pK-conjugate of OVA is about 100 fold more efficient in stimulating antigen presentation than unconjugated OVA. (See Figs. 2A-C)

A characteristic of DC, a potent subset of APC, is their ability to trigger *in vivo* responses of naive CD8⁺ cytotoxic T-lymphocytes (CTL), after being pulsed with antigen (Ridge et al. 1998 Nature 393:474). As exemplified herein, DC are able to induce a CTL response in mammals not previously exposed to a soluble protein antigen when the antigen was modified by conjugation with pEA/pK, but not when an unmodified form of the antigen was used to pulse the DC.

The superior immunogenicity of the OVA antigen modified by conjugation to the pK peptide (SEQ ID NO: 1) and the peptide (SEQ ID NO: 2); as determined by stimulation of a CD8⁺-mediated immune response is further detailed in Example 2. The Example describes the increased survival of mice bearing OVA-expressing tumor cells when injected with DC pre-pulsed with pEA/pK-conjugated OVA prior to injection relative to the response to DC pre-pulsed with OVA alone. (see Fig 6A-B).

It will be understood that any of a number of methods may be used to pulse APC with antigen, to make them effective to present antigen in the context of MHC I. The experiments detailed herein demonstrate that activation of DC by exposure to modified antigenic peptides facilitates processing of the peptides through the "endogenous" class I pathway such that they are presented in association with MHC class I molecules, and accordingly are able to activate CD8⁺. CTL.

### VI. Compositions and methods for immunotherapy and cancer therapy

One of the useful features of the antigenic compositions of the present invention is that they are able to more effectively present antigen for the induction of both CD8⁺ CTL-mediated as well as CD4⁺ Th cell proliferative responses than APC which have not been so modified.

As such, the antigenic compositions of the present invention are universally useful and can be employed in a wide range of immunotherapeutic, immunoprophylactic and cancer therapeutic applications involving generation of primary and secondary immune responses.

The invention also provides modified soluble protein antigens presented in the context of MHC Class I.

In a preferred embodiment, immunization with the modified soluble protein antigens of the invention results in an MHC Class I-mediated cellular immune response to a soluble protein antigen which would not elicit a cellular immune response if provided in an unmodified form. In a further preferred embodiment, immunization with such a modified soluble protein antigen results in an MHC Class I-mediated cellular immune response which is greater in magnitude and accordingly provides greater protection than a cellular immune response to the same antigen if provided in an unmodified form.

In a preferred embodiment, the present invention provides an antigenic composition comprising modified antigens and APCs, as described above, which is able to more effectively induce T-cell responses, than an antigenic composition comprising antigens which have not been so modified.

An antigenic composition comprising the combination of modified antigens and APCs yields a novel antigenic composition which finds utility in immunotherapy of a mammal and may function as a vaccine.

Also described is a method of immunizing a subject against an antigen, *e.g.*, a known cancer or pathogen-specific antigen or immunogen. The method includes pulsing APC or DC with a selected antigen or immunogen wherein the antigen or immunogen has an added peptidic sequence (which facilitates entry of the antigen or immunogen into APC or DC), by exposing the APC or DC to the modified antigen in a manner effective to induce cell-surface presentation of one or more peptide antigens specific to the antigen or immunogen and returning the pulsed APC or DC to the subject.
The exposing step can be achieved either *in vitro* (*ex vivo*) or in vivo. For example, the modified antigen may be directly injected into a subject or the APC or DC of the subject exposed to the modified antigen *in vitro* (*ex vivo*), with the stimulated APC or DC returned to the subject.

In a further preferred embodiment the modified soluble protein antigen in the antigenic composition is a cancer-specific antigen or tumor antigen. Exemplary cancer-specific or tumor antigens known to those of skill in the art include, but are not limited to, PAP, HER2, MART-1, MAGE, BAGE, and GAGE.

In a related aspect, the cancer-specific or tumor antigen is modified by covalent conjugation to a peptide selected from the group consisting of the pK, pEA, HA, tandem pEA/pK, tandem HA/pK peptides, peptides comprising lysine and arginine residues and peptides comprising repeating subunits presented as SEQ ID NO:1, SEQ ID NO:2, SEQ ID NO:3, SEQ ID NO:4, SEQ ID NO:5, SEQ ID NO:6, SEQ ID NO:7 and the sequences designated as sequence number 8 and sequence number 9, respectively:

In a preferred embodiment, the cancer-specific or tumor antigen is modified by covalent conjugation to the pK peptide (SEQ ID NO: 1) and the pEA peptide (SEQ ID NO: 2).

Such antigenic composition comprising modified antigens alone or in combination with modified APC can be used, for example, in direct *in vivo* administration, *ex vivo* somatic therapy, *in vivo* implantable devices and *ex vivo* extracorporeal devices.

Further described is a method of immunizing a subject against a tumor or pathogen having a known tumor- or pathogen-specific antigen which includes the steps of; obtaining a population of APC or DC from a blood sample taken from a patient (subject), exposing the APC or DC to a modified tumor- or pathogen-specific antigen *in vitro* in a manner effective to induce cell-surface presentation of one or more peptide antigens against which an immune response is desired; and returning the pulsed APC or DC to the subject.

From the foregoing, it will be appreciated that the invention provides compositions having the unique feature that processing of soluble protein antigens occurs through the MHC class I, as opposed to class II, pathway.

Although the invention has been described with reference to specific methods and embodiments, it is appreciated that various modifications and changes may be made without departing from the invention.

### Example 1

### Evaluation of in vitro presentation of OVA-peptide conjugates

The IL-2 secreting mouse T cell hybridoma B3Z, which responds to the mouse MHC class I (H2-K^{b}) bound OVA-derived peptide SIINFEKL (OVA₂₅₇₋₂₆₄; Jameson et al., J. Exp. Med. 177: 1541, 1993), was used to evaluate the presentation efficacy of various OVA-peptide conjugates by the thymoma cell line EL-4.

Grade VI (99% pure) chicken ovalbumin (OVA) purchased from Sigma (St. Louis, MO), was used either in its native or conjugated form. Peptide conjugates OVA-pEA, OVA-pK, OVA-HA, OVA-HA/pK and OVA-pEA/pK were prepared as custom reagents by a commercial manufacturer (Biosynthesis, Inc , Lewisville, TX) according to standard methods. E.G7-OVA is the EL-4 cell line transfected with full length OVA cDNA (Brossart et al., J. Immunol. 158: 3270, 1997).

Briefly, peptides Cys-Lys-Lys-Lys-Lys-Lys-Lys-Lys-Lys-Lys-Lys-Lys-Lys-Lys-Lys-Lys-Lys-Lys-Lys-Lys-Lys ("pK", SEQ ID NO: 1), Cys-Glu-Ala-Ala-Ala-Ala-Ala-Glu-Ala-Ala-Ala-Ala-Ala-Glu-Ala-Ala-Ala-Ala-Ala-Glu-Ala-Ala-Ala-Ala-Ala ("pEA", SEQ ID NO: 2) and Cys-Gly-Leu-Phe-Gly-Ala-Ile-Ala-Gly-Phe-Ile-Glu-Asn-Gly-Trp-Glu-Gly-Met-Ile-Asp-Gly-Trp-Tyr-Gly ("HA", SEQ ID NO: 3) were synthesized by Fmoc chemistry on SPAR 50 polyamide resins. Peptides were purified by HPLC and conjugated to OVA with sulfo-MBS (*m*-maleimidobenzoyl-N-hydroxysulohosuccinimide ester).

For the experiment shown in Fig. 1, cell cultures were maintained in DMEM medium supplemented with 10% FCS, 292 µg/ml of L-glutamine, 100 U/ml of penicillin, 100 U/ml of streptomycin and 55 µM of 2-ME (Gibco, Grand Island, NY) at 37°C in a humidified atmosphere containing 10% CO2. Mouse thymoma cells EL-4 were pulsed overnight at 37°C with indicated antigens by co-culturing 1 x 10⁶ cells with 2 µM of each antigen. Subsequently, pulsed APC were washed and gamma-irradiated (30 Gy), and an IL-2 secretion assay was performed as described previously (Kruisbeek, 1998, in Coligan et al. (eds.) Current Protocols in Immunology Wiley, New York, NY, 1:3.14). Namely, 1 x 10⁵ B3Z hybridoma cells were cultured in 0.2 ml microwells in the presence of a given number of APC (as shown in Fig. 1). One day later, culture supernatants were harvested and tested for their ability to support the proliferation of 10⁴ HT-2 cells (an IL-2 dependent cell line) for 24 h. Cell growth was measured by [³H]thymidine incorporation during the final 6 h culture period.

When EL-4 cells were pulsed with antigen at a concentration equivalent to the nonstimulatory (*i.e.,* suboptimal) dose of native OVA, neither OVA, OVA-pEA, OVA-HA nor OVA-pK stimulated B3Z above the background level, OVA conjugated to the pK peptide (SEQ ID NO: 1) and the pEA peptide (SEQ ID NO: 2) and OVA conjugated to the pK peptide and the HA peptide (SEQ ID NO: 3) triggered positive responses. Among the two, the OVA-pK/pEA conjugate was more potent. See Table 1, below, for exemplary peptidic sequences for addition to soluble protein antigens in the methods and compositions of the invention.

Tissue cultures for the experiments shown in Figs 2-5 were maintained in IMDM medium supplemented with 10% FCS, 2 mM L-glutamine, 0.1 mg/ml kanamycin sulfate and 3 x 10⁻⁵ M 2-ME (Gibco, Grand Island, NY) at 37°C in a humidified atmosphere containing 5% CO₂ (tissue culture incubator). 10⁵ hybridoma cells were cultured in 0.2-ml microwells in the presence of 3 x 10⁴ Ag-pulsed APC. One day later, culture supernatants were harvested and tested at 50% concentration for their ability to support the proliferation of 10⁴ HT-2 cells for 24 h (measured by incorporation of [³H]thymidine present during the final 6 h culture period).

**Table 1. Exemplary Peptidic Sequences For Addition To Soluble Protein Antigens.**

| | **Description** | **Designation** |
|---|---|---|
| ref. peptide | | SEQ ID NO:1 (PK) |
| | | SEQ ID NO:2 (pEA) |
| | | SEQ ID NO:3 (HA) |
| | | SEQ ID NO:4 (tandem pEA/pK conjugate) |
| | | SEQ ID NO:5 (tandem HA/pK conjugate) |
| ref. peptide | CYS-X-X-X-X-X-X-X-X-X-X-X-X-X-X-X-X-X-X-X-X, wherein X= arg or lys | SEQ ID NO:6 |
| ref. peptide | X-X-X-X-X-X-X-X-X-X-X-X-X-X-X-X-X-X-X-X, wherein X= arg or lys | SEQ ID NO:7 |
| ref. peptide | CYS-[X-Y-Y-Y-Y-Y]ₙ; wherein X= glu or asp, Y = ala, leu, ile, phe, 2ly, cys, met or val and n is greater than or equal to 3 | sequence number 8 |
| ref. peptide | [X-Y-Y-Y-Y-Y]ₙ; wherein X = glu or asp, Y = ala, leu, ile, phe, gly, cys, met or val and n is greater than or equal to 3 | sequence number 9 |

Analysis of B3Z responses to EL-4 APC pre-pulsed with a wide range of antigen concentrations indicates that pEA/pK-conjugate of OVA is about 100 fold more efficient in stimulating B3Z than OVA itself. (See Figs. 2A-C)

The above evidence appears to reflect improved penetration of pEA/pK-conjugated OVA into the class 1-dependent Ag processing and presentation pathway. An additional experiment was conducted in order to eliminate the possibility that Ag-derived peptides in the conjugate (which do not require internalization or processing), were responsible for the observed improvement in B3Z responses. Cells were cultured as described above and EL-4 cells were fixed with 0.025% glutaraldehyde (Fluka, Buchs, Switzerland) prior to Ag pulsing. When APC were fixed with glutaraldehyde prior to Ag pulsing thereby preventing Ag internalization and processing, fixed EL-4 cells were observed to present the immunogenic peptide SIINFEKL to B3Z in a stimulatory fashion, while their capability to present OVA-pEA/pK was completely lost upon fixation (see Fig. 3). SIINFEKL is an OVA- derived peptide (OVA₂₅₇₋₂₆₄), recognized by the T cell hybridoma B3Z. (Jameson et al., 1993, J. Exp. Med. 177: 1541) A residual (approximately 7%) B3Z response to fixed EL-4 pulsed with unmodified OVA was observed indicating that the latter, though 99% pure (Sigma, St. Louis, MO) may contain a minor fraction of degradation product(s), which are removed upon conjugation to pEA/pK.

### Example 2

### Evaluation of in vivo presentation or OVA-pEA/pK conjugate

In general, T cell hybridomas represent highly activated memory T cells, with an activation threshold significantly lower then that required to trigger naive T cells (Ridge et al., 1998, Nature 393: 474). It has been found that only highly activated DC are capable of triggering effective CTL responses in animals not previously exposed to the antigen. Accordingly, we immunized virgin laboratory mice with Ag-pulsed DC in order to examine whether OVA conjugated to pK and pEA could elicit an effective CTL response.

Dendritic cells were pulsed by 16h co-culture with either OVA (5 µM) or OVA-pEA/pK (0.5 µM), washed two times and injected into mice. Female C57BL/6 mice were either left untreated, or were immunized with two intraperitoneal (i.p.) injections of 5 x 10⁵ Ag-pulsed cells in 0.1 ml phosphate buffered saline (PBS) at a two week interval. Two weeks after the last *in vivo* immunization, splenocytes were pooled from 2 mice of each group and restimulated *in vitro* by co-culturing them at a density of 4 x 10⁶ cells/ml with gamma-irradiated (200 Gy) E.G7-OVA at 5 x 10⁵ cells/ml, in the presence of the recombinant human IL-2 (10 u/ml; Genzyme, Cambridge, MA). Six days later the cultures were tested for their ability to lyse ⁵¹Cr-labeled E.G7-OVA and EL-4 target cells in a standard cell mediated cytotoxicity assay (Wunderlich and Shearer 1998, in Coligan et al. (eds.) Current Protocols in Immunology Wiley, New York, NY, 1:3.11).

As shown in Fig. 4, only mice primed with DC previously pulsed with OVA conjugated to pEA and pK generated significant CTL responses against OVA-transfected target cells (E.G7-OVA), while the corresponding responses of mice immunized with OVA-pulsed DC were substantially lower.

Tumor specific CTL constitute an important effector limb of the anti-tumor immune response (Greenberg, 1991, Adv. Immunol. 49: 281). Accordingly, the effect of immunization with OVA-pEA/pK-pulsed DC on suppression of *in vivo* growth of OVA-expressing autologous tumors was evaluated.

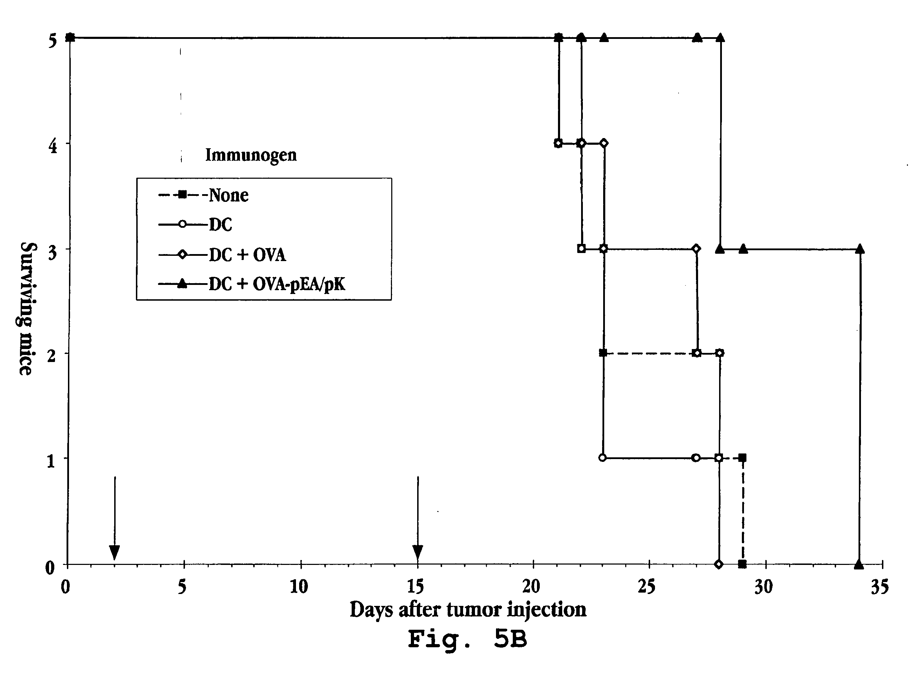

Twenty 8-week old randomized female C57BL/6 mice were each injected i.p. with E.G7-OVA cells in 0.1 ml PBS (Fig. 5A: 25 x 10⁶ cells per mouse; Fig. 5B: 2 x 10⁶ cells per mouse). Two days and again 2 weeks later the mice received i.p. injections of either unpulsed DC, OVA-pulsed DC, OVA-pEA/pK-pulsed DC (5 x 10⁵ cells per 0.1 ml injection), or PBS. Mice were monitored daily and their survival was recorded.

As shown in Fig. 5, treatment with DC pulsed with OVA conjugated with pK and pEA exhibited a notable therapeutic effect, considerably prolonging the survival of tumor-bearing mice (statistical significance according to Student's *t*-test: *p*<0.01).

Although the invention has been described with reference to specific methods and embodiments, it will be appreciated that various modifications and changes may be made without departing from the invention.

## Claims

1. An antigen composition which elicits an enhanced cytotoxic T cell response in the context of a major histocompatibility complex class I molecule (MHC class I), comprising a soluble protein antigen (i) chemically linked or (ii) conjugated by expression of a continuous nucleic acid coding sequence to one or more added peptidic sequences selected from the group consisting of SEQ ID NO:2, SEQ ID NO:3, SEQ ID NO:4, and SEQ ID NO:5 and a sequence presented as CYS-[X-Y-Y-Y-Y-Y]ₙ; wherein X = glu or asp, Y = ala, leu, ile, phe, gly, cys, met or val and n is greater than or equal to 3 or [X-Y-Y-Y-Y-Y]ₙ; wherein X = glu or asp, Y = ala, leu, ile, phe, gly, cys, met or val and n is greater than or equal to 3, wherein said added peptidic sequence facilitates entry of said antigen into antigen presenting cells (APC), and wherein said antigen composition activates T cells to produce a cytotoxic cellular immune response against said antigen, at a T cell level that is higher than that produced by such APC stimulated by the antigen alone.

2. The antigen composition of claim 1 for use in ummunizing a subject against a tumor or pathogen where said antigen is specific to the tumor or pathogen.

3. The antigen composition of claim 1, wherein said one or more added peptidic sequences are chemically linked to said antigen.

4. The antigen composition of claim 1, wherein said antigen composition is a fusion protein produced by translation of a continuous nucleotide coding sequence.

## Patentansprüche

1. Antigenzusammensetzung, die eine verstärkte cytotoxische T-Zell-Antwort im Zusammenhang mit einem Haupthistokompatibilitätskomplex Klasse-I-Molekül (MHC I) hervorruft, umfassend ein lösliches Protein-Antigen, das (i) chemisch verbunden oder (ii) durch Expression einer kontinuierlichen, codierenden Nucleinsäuresequenz konjugiert ist mit einer oder mehreren hinzugefügten Peptidsequenz(en), ausgewählt aus der Gruppe bestehend aus SEQ ID NO: 2, SEQ ID NO: 3, SEQ ID NO: 4 und SEQ ID NO: 5 und einer Sequenz präsentiert als CYS-[X-Y-Y-Y-Y-Y]ₙ, wobei X = Glu oder Asp, Y = Ala, Leu, Ile, Phe, Gly, Cys, Met oder Val und n größer oder gleich 3 ist, oder [X-Y-Y-Y-Y-Y]ₙ, wobei X = Glu oder Asp, Y = Ala, Leu, Ile, Phe, Gly, Cys, Met oder Val und n größer oder gleich 3 ist, wobei die hinzugefügte Peptidsequenz den Eintritt des Antigens in antigenpräsentierende Zellen (APC) erleichtert, und wobei die Antigenzusammensetzung T-Zellen aktiviert, eine cytotoxische zelluläre Immunantwort gegen das Antigen zu produzieren, bei einem T-Zell-Spiegel, der höher ist als der, der durch solche APC produziert wird, die durch das Antigen allein stimuliert werden.

2. Antigenzusammensetzung nach Anspruch 1 zur Verwendung in der Immunisierung eines Individuums gegen einen Tumor oder ein Pathogen, wobei das Antigen spezifisch für den Tumor oder das Pathogen ist.

3. Antigenzusammensetzung nach Anspruch 1, wobei die eine oder die mehreren hinzugefügten Peptidsequenz(en) chemisch mit dem Antigen verbunden ist/sind.

4. Antigenzusammensetzung nach Anspruch 1, wobei die Antigenzusammensetzung ein Fusionsprotein ist, das durch Translation einer kontinuierlichen, codierenden Nucleotidsequenz produziert wird.

## Revendications

1. Composition d'antigène qui provoque une réponse des cellules T cytotoxiques augmentée dans le contexte d'une molécule de complexe majeur d'histocompatibilité de classe I (CMH de classe I), comprenant un antigène protéique soluble (i) lié chimiquement ou (ii) conjugué par expression d'une séquence codante d'acide nucléique continue à une ou plusieurs séquences peptidiques ajoutées choisies dans le groupe constitué de SEQ ID NO :2, SEQ ID NO :3, SEQ ID NO :4, et SEQ ID NO :5, et une séquence présentée comme CYS-[X-Y-Y-Y-Y-Y]ₙ ; dans laquelle X est Glu ou Asp, Y est Ala, Leu, Ile, Phe, Gly, Cys, Met ou Val et n est supérieur ou égal à 3 ou [X-Y-Y-Y-Y-Y]ₙ; dans laquelle X est Glu ou Asp, Y est Ala, Leu, Ile, Phe, Gly, Cys, Met ou Val et n est supérieur ou égal à 3, ladite séquence peptidique ajoutée facilitant l'entrée dudit antigène dans des cellules présentatrices d'antigène (CPA), et dans laquelle ladite composition d'antigène active les cellules T pour produire une réponse immunitaire cytotoxique cellulaire contre ledit antigène, à un niveau de cellule T qui est plus élevé que celui produit par une telle CPA stimulée par l'antigène seul.

2. Composition d'antigène selon la revendication 1 pour une utilisation pour l'immunisation d'un sujet contre une tumeur ou un pathogène, dans laquelle ledit antigène est spécifique de la tumeur ou du pathogène.

3. Composition d'antigène selon la revendication 1, dans laquelle lesdites une ou plusieurs séquences peptidiques ajoutées sont liées chimiquement audit antigène.

4. Composition d'antigène selon la revendication 1, dans laquelle ladite composition d'antigène est une protéine de fusion produite par traduction d'une séquence codante nucléotidique continue.
